# EUROPEAN PATENT SPECIFICATION

(11) **EP 2 152 343 B1**
(45) Date of publication and mention of the grant of the patent: **25.07.2012**
(21) Application number: 08750803.2
(22) Date of filing: 04.06.2008
(51) Int. Cl.: A61M 16/08, A61M 16/20, A61M 39/26

(54) **IMPROVEMENTS RELATING TO RESPIRATORY CONNECTORS**
VERBESSERUNGEN IM ZUSAMMENHANG MIT ATEMKONNEKTOREN
PERFECTIONNEMENTS APPORTÉS À DES CONNECTEURS RESPIRATOIRES

(30) Priority: 04.06.2007 GB 0710566
(43) Date of publication of application: 17.02.2010
(73) Proprietor: Intersurgical AG, Vaduz (LI)
(72) Inventor: BOOTH, Christopher, Chester Cheshire CH3 8AB (GB)
(74) Representative: Adamson Jones
(86) International application number: PCT/GB2008/050408
(87) International publication number: WO 2008/149151

(56) References cited:
- WO-A-2004/098689
- US-A- 4 951 661
- US-A1- 2002 162 554

## Description

This invention relates to respiratory connectors, and in particular connectors for operably connecting a nebuliser to a breathing circuit.

Nebulisers are generally used in breathing circuits to introduce a fine mist of medicament into gases that are to be inhaled by the patient. Conventionally, a nebuliser is connected to a breathing circuit by means of a T-piece connector that forms part of the breathing circuit. In particular, the T-piece connector is generally connected in-line within the breathing circuit, a branched limb of the T-piece connector forming an access port that is adapted to releasably engage with a nebuliser.

T-piece connectors have been developed with a valve mechanism for enabling fluid communication between the access port of the T-piece connector and the breathing circuit when the nebuliser is connected, and sealing the access port from the breathing circuit when the nebuliser is disconnected. In particular, conventional T-piece connectors typically include a valve member having a closed configuration in which the valve member seals a valve port from the fluid conduit, and an open configuration in which the valve member enables fluid communication between the valve port and the fluid conduit, the valve member being resiliently biased into its closed configuration. The document US-A-4,951,661 discloses one such connected with a valve mechanism.

However, conventional valve mechanisms in T-piece connectors typically restrict the cross-sectional area of the valve port significantly, and hence can adversely affect the efficiency of drug delivery to the breathing circuit. Furthermore, conventional valve mechanisms can be of complex construction, often including a significant number of separate components, and hence manufacturing costs for such valve mechanisms can be high. The seal provided by conventional valve mechanisms can also be less than satisfactory when the T-piece connector is in its sealed configuration.

There has now been devised an improved respiratory connector which overcomes or substantially mitigates the above-mentioned and/or other disadvantages associated with the prior art.

According to the invention, there is provided a respiratory connector comprising first and second limbs defining a fluid conduit adapted for incorporation into a breathing circuit, a third limb defining a valve port adapted for connection to a respiratory component, a valve member movable relative to the valve port between an open configuration in which the valve member enables fluid communication between the valve port and the fluid conduit, and a closed configuration in which the valve member seals the valve port from the fluid conduit, and a fourth limb that houses means for resiliently biasing the valve member into the closed configuration.

The respiratory connector according to the invention is advantageous principally because the resilient means is housed within a fourth limb of the respiratory connector, rather than within the valve port or the fluid conduit. The restriction of the cross-sectional area of the valve port and/or the fluid conduit caused by the presence of the valve mechanism may therefore be significantly reduced. The present invention is particularly advantageous for connecting a nebuliser to a breathing circuit, such that gas and entrained medicament supplied by the nebuliser mix with gases flowing through the fluid conduit and are therefore delivered by the breathing circuit to the patient. The present invention may therefore increase the efficiency of drug delivery to the breathing circuit relative to conventional respiratory connectors including valve mechanisms.

The resilient means will typically take the form of a helical spring, but other resilient members could be used, such as members formed of an elastomeric material. The fourth limb that houses the resilient means preferably extends from an opening in a part of the side wall of the fluid conduit that is substantially opposite that part from which the third limb defining the valve port extends. In particular, the third and fourth limbs are preferably aligned, most preferably co-axially.

The valve member is preferably received within the third limb defining the valve port, preferably with a close fit, so that it is slidable relative to the valve port. Most preferably, the valve member is also received within the fourth limb housing the resilient means, preferably with a close fit, so that it is slidable relative to both the valve port and the fourth limb. Most preferably, the resilient means is interposed between an end wall of the fourth limb, and an end surface of the valve member.

The valve member preferably includes a sealing member that cooperates with a sealing surface of the valve port so as to seal the valve port from the fluid conduit in the closed configuration. The sealing member is preferably situated externally of the fluid conduit, so as not to impede flow through the fluid conduit, in the closed configuration. The sealing member may be adapted to partition the interior volume of the valve port so as to prevent flow of gas through the valve port into the fluid conduit, in the closed configuration. However, the sealing member is preferably adapted to occlude an opening in the side wall of the fluid conduit from which the valve port extends so as to prevent flow of gas between the valve port and the fluid conduit, in the closed configuration.

The sealing member is preferably generally planar in form, and most preferably has the form of a disc. The major surfaces of the sealing member are preferably orientated transversely to the longitudinal axis of valve port. Furthermore, the sealing surface of the valve port, with which the sealing member engages in the closed configuration, is preferably inclined relative to the longitudinal axis of the valve port and hence also the direction of movement of the valve member.

The sealing member may be located either within the fluid conduit, or alternatively within the chamber defined by the fourth limb of the respiratory component, in the open configuration. Where the sealing member is located within the fluid conduit in the open configuration, the major surfaces of the sealing member are preferably orientated parallel to the longitudinal axis of fluid conduit, so as to minimise the reduction in cross-sectional area of the fluid conduit caused by the presence of the sealing member.

The valve member is preferably adapted to be moved from the closed configuration to the open configuration by engagement of the respiratory component with a tubular connector of the valve port. In particular, the valve member preferably includes an actuator member adapted to be engaged by the tubular connector of the respiratory component, during its engagement with the tubular connector of the valve port, such that the valve member is moved from the closed configuration to the open configuration. The actuator member is preferably slidably mounted within the valve port.

In presently preferred embodiments, the valve member includes a tubular actuator member. The actuator member may be adapted to engage an interior or exterior surface of the tubular connector of the respiratory component with an interference fit. However, in presently preferred embodiments, the actuator member is adapted to abut an interior or exterior surface of the tubular connector of the respiratory component. In any case, the actuator member is preferably adapted to be urged from the closed configuration to the open configuration by the tubular connector of the respiratory component, during engagement of the respiratory component with the respiratory connector. The actuator member may or may not project from the valve port, depending upon the manner in which the respiratory component is adapted to engage the actuator member.

The valve member is preferably adapted so that, in the open configuration, the sealing member does not prevent flow of gas between the respiratory component and the fluid conduit. In presently preferred embodiments, the sealing member is separated from the actuator member by one or more separator members that define one or more flow openings between the sealing member and the actuator member. The valve member is preferably adapted so that flow paths are defined through the one or more flow openings, in the open configuration, between the respiratory component and the fluid conduit. In addition, the one or more separator members are preferably disposed within one or more recesses in the interior surface of the fluid conduit in the open configuration, so as to not impede flow of gas through the fluid conduit.

In presently preferred embodiments, where the respiratory connector is adapted for use with a respiratory component having a male tubular connector, a tubular connector of the valve port is preferably adapted to engage an exterior surface of the male tubular connector with an interference fit, and the actuator member is preferably adapted to abut formations on the interior surface of the male tubular connector, and then be urged along the valve port from the closed configuration to the open configuration. In presently preferred embodiments, where the respiratory connector is adapted for use with a respiratory component having a female tubular connector, a tubular connector of the valve port is preferably adapted to engage an interior surface of the female tubular connector with an interference fit, and the actuator member is preferably adapted to abut formations on the interior surface of the female tubular connector, and then be urged along the valve port from the closed configuration to the open configuration.

The valve member preferably includes an end portion that cooperates with the resilient means housed by the fourth limb, and preferably extends into the fourth limb in at least the open configuration, and most preferably both the open and closed configurations. The end portion of the valve member is preferably slidably mounted within the fourth limb, and preferably includes an operative surface adapted to be engaged by the resilient means. The valve member preferably includes one or more separator members that separate the sealing member from the end portion of the valve member. The end portion of the valve member is preferably located within the opening in the side wall of the fluid conduit, from which the fourth limb extends, in the closed configuration, and is preferably located within the fourth limb in the open configuration. The one or more separator members are preferably disposed within one or more recesses in the interior surface of the fluid conduit in open configuration, so as to not impede flow of gas through the fluid conduit.

The respiratory connector is preferably assembled from one or more components formed of a suitable plastics material, for example polypropylene. The connector may also include an elastomeric seal component, which is preferably formed of a suitable thermoplastic elastomer (TPE). In this case, at least one component of the respiratory connector is preferably manufactured using a two-shot injection moulding process.

The end portions of the first and second limbs preferably define an inlet port and an outlet port of the fluid conduit. The inlet and outlet ports both preferably take the form of conventional tubular connectors, for example 22mm tubular connectors. The fluid conduit is preferably of substantially constant cross-section, and preferably extends along a generally linear central axis. However, the respiratory connector may include one or more recesses in its interior surface, which form one or more enlarged parts of the fluid conduit. The one or more recesses preferably accommodate one or more parts of the valve member so that those one or more parts are situated externally of the linear flow paths through the fluid conduit.

The valve port preferably extends from an opening in the side wall of fluid conduit, and preferably includes a tubular connector at its outer end, the tubular connector being adapted for connection to a corresponding tubular connector of the respiratory component. The tubular connector of the valve port preferably has the form of a conventional male or female tubular connector, with an operative surface adapted to engage the corresponding tubular connector of the respiratory component with an interference fit. Most preferably, the operative surface is inclined relative to the longitudinal axis of the tubular connector, in order to aid engagement between the tubular connectors.

Where the respiratory component is a nebuliser, the valve port will typically extend downwardly from the fluid conduit during use. In this case, the respiratory connector preferably includes one or more barriers at the lower part of the fluid conduit for impeding flow of any liquid within the breathing circuit into the valve port.

The valve port is preferably defined by a separate component, ie a valve port component, to the component that defines the fluid conduit and includes the inlet/outlet ports, ie the connection component. The valve port component preferably comprises a mounting for connecting to the connection component. In presently preferred embodiments, the valve port component and the connection component include corresponding connecting formations, which preferably engage one another with a snap-fit.

As discussed above, the respiratory connector may include an elastomeric seal component. This seal component preferably provides a sealing surface with which the valve member engages in the closed configuration, thereby sealing the valve port from the fluid conduit. Most preferably, this sealing surface is annular in form. In addition, the seal component may define a sealing surface for providing a hermetic seal between the valve port component and the connection component.

Preferred embodiments of the invention will now be described in greater detail, by way of illustration only, with reference to the accompanying drawings, in which
Figure 1 is a perspective view of a first embodiment of a respiratory connector according to the invention;
Figure 2 is an exploded perspective view of the respiratory connector of Figure 1;
Figure 3 is a perspective view of the respiratory connector of Figure 1, partially cut-away, in its closed configuration;
Figure 4 is a fragmentary, cross-sectional view of the respiratory connector of Figure 1 in its closed configuration;
Figure 5 is a perspective view of the respiratory connector of Figure 1, partially cut-away, in its open configuration;
Figure 6 is a fragmentary, cross-sectional view of the respiratory connector of Figure 1 in its open configuration; and
Figure 7 is a perspective view of a second embodiment of a respiratory connector according to the invention.

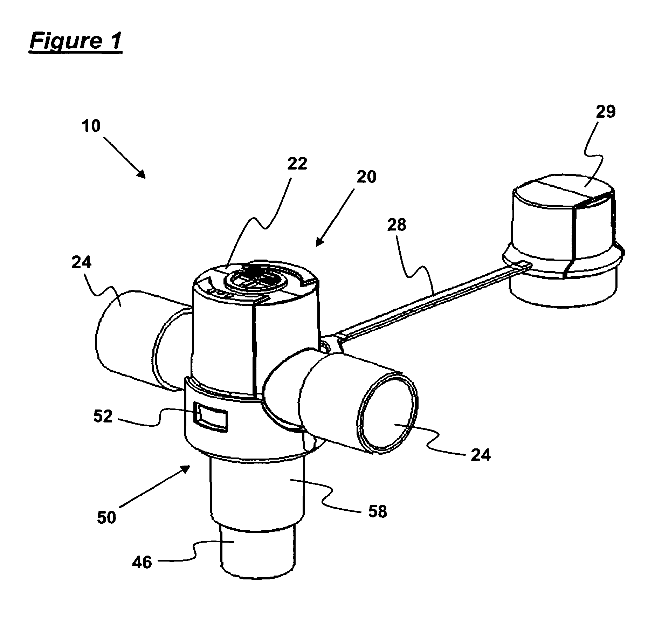

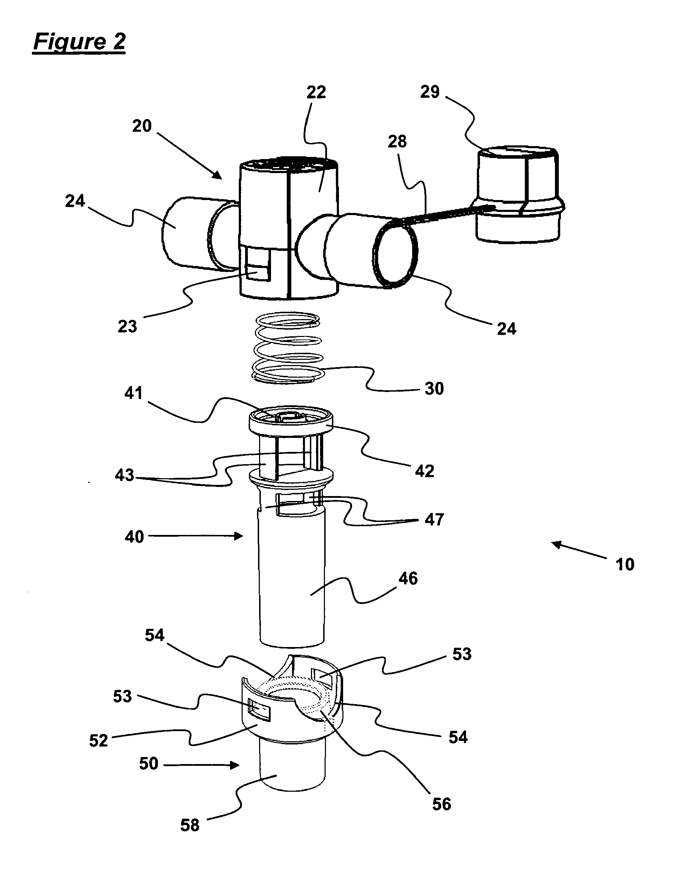

Figures 1 and 2 show a first embodiment of a respiratory connector according to the invention, which is generally designated 10. This respiratory connector 10 is adapted to connect to a respiratory component, such as a nebuliser, having a female tubular connector. The respiratory connector 10 is assembled from a connection component 20, a valve spring 30, a valve member 40 and a valve port component 50, as shown most clearly in Figure 2.

The connection component 20, the valve member 40 and the valve port component 50 are formed of a suitable plastics material, such as polypropylene, by injection moulding. The plastics material will be suitably strong and relatively rigid in form. However, the valve port component 50 also incorporates a seal component 56 formed of a thermoplastic elastomeric material. The valve port component 50 is therefore manufactured using a so-called two-shot injection moulding process. In particular, the valve port component 50 is injection moulded in a suitably strong and relatively rigid plastics material, such as polypropylene, and then a thermoplastic elastomeric material is injection moulded onto an external surface of the valve port component 50, while it is incompletely cured, so as to form the seal component 56. Since the seal component 56 is moulded directly onto the valve port component 50, these components 50,56 become bonded together.

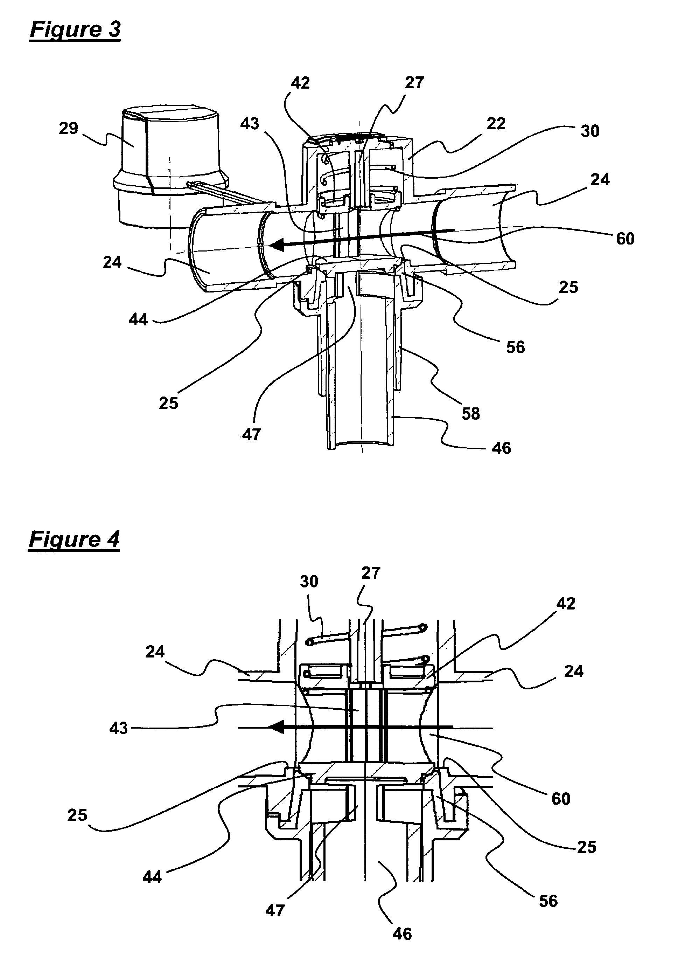

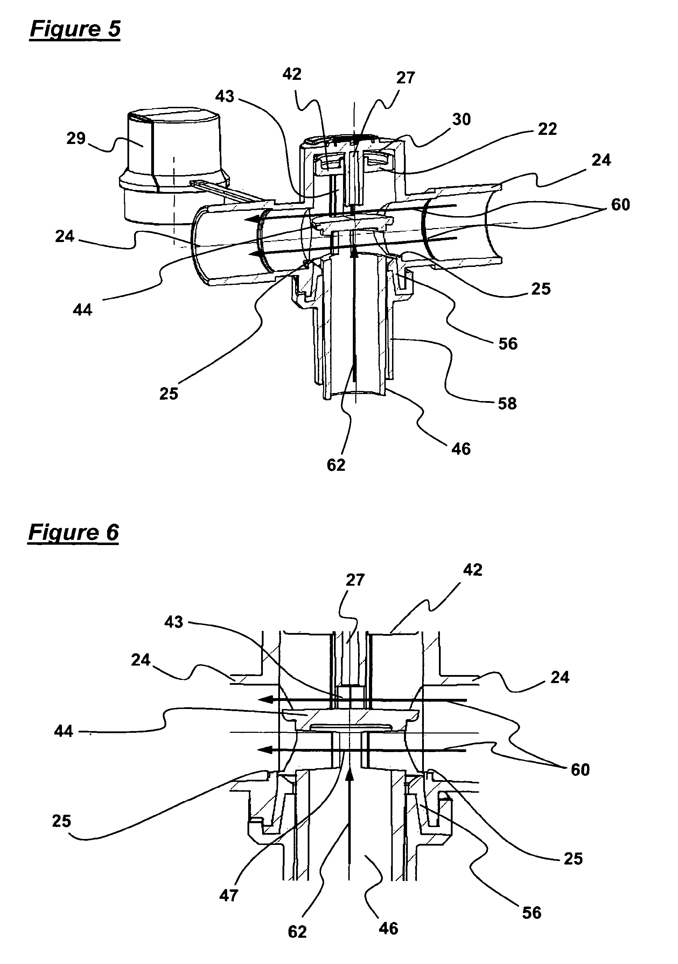

The connection component 20 comprises a central cylindrical housing 22 and diametrically opposed inlet/outlet ports 24 that extend perpendicularly from the cylindrical housing 22. The cylindrical housing 22 and the inlet/outlet ports 24 together define a fluid conduit though the connection component 20. The inlet/outlet ports 24 each have an end portion that has the form of a 22mm tubular connector, with a tapered operative surface, such that the fluid conduit of the connection component 20 may be incorporated, in use, into an otherwise conventional breathing circuit (not shown in the Figures). The cylindrical housing 22 of the connection component 20 has a diameter that is greater than the diameters of the adjacent portions of the inlet/outlet ports 24, such that a recess is defined on either side of the fluid conduit that extends through the connection component 20. In addition, as shown most clearly in Figures 3 and 4, an upstanding step 25 is formed across the lower end (as shown in Figures 3 and 5, and as typically orientated during use) of each of the inlet/outlet ports 24, at the periphery of the cylindrical housing 22. These upstanding steps 25 provide a barrier for reducing the risk of liquids within the breathing circuit flowing through the valve port component 50 into the connected respiratory component, such as a nebuliser.

The valve member 40 of the respiratory connector 10 is slidably mounted within the cylindrical housing 22 of the connection component 20, and also within the valve port component 50. The valve member 40 comprises an upper sealing disc 42, a lower sealing disc 44 and a tubular actuator member 46, which are aligned along a longitudinal axis of the valve member 40. The lower sealing disc 44 is separated from the upper end of the tubular actuator member 46 (as viewed in Figure 2) by a first pair of diametrically opposed struts 47, and the upper sealing disc 42 is separated from the lower sealing disc 44 by a second pair of diametrically opposed struts 43. The lower end of the tubular actuator member 46 projects from the lower end of the valve port.

The tubular actuator member 46 has a tapered external surface, an end portion of which is adapted to abut formations on an interior surface of an otherwise conventional female 22mm tubular connector of a respiratory component, such as a nebuliser, that is engaged, in use, with the valve port component 50, as described in more detail below. The formations may have the form of one or more projections, or a shoulder, on the interior surface of the tubular connector. The upper sealing disc 42 of the valve member 40 includes a central opening 41, and a pair of upstanding skirts on the upper surface of the upper sealing disc 42, one at the periphery of the upper sealing disc 42 and one at the periphery of the central opening 41. This pair of skirts defines a depression within which one end of the valve spring 30 is located.

A cylindrical spigot 27 extends downwardly from the interior surface of the upper end of the cylindrical housing 22 (as shown in Figures 3 and 5), and this cylindrical spigot 27 is slidably received within the opening 41 of the upper sealing disc 42. A chamber is therefore defined in an upper part of the cylindrical housing, above the upper sealing disc 42, within which the valve spring 30 of the respiratory connector 10 is housed. The valve spring 30 therefore abuts an interior surface of the upper end of the cylindrical housing 22, and the central spigot 27 extends along a central axis of the valve spring 30.

The valve member 40 is slidably mounted within the cylindrical housing 22 of the connection component 20, and the valve port component 50, such that it is movable between a closed configuration in which the valve member 40 seals the valve port from the fluid conduit, and an open configuration in which the valve member 40 enables fluid communication between the valve port and the fluid conduit. Furthermore the valve spring 30 resiliently biases the valve member 40 into its closed configuration. These configurations are described in more detail below in relation to Figures 3 to 6.

The valve port component 50 comprises an upper cylindrical part that defines a mounting 52 adapted for connection with the lower end of the central cylindrical housing 22 of the connection component 20, and a lower cylindrical part of reduced diameter that defines a tubular connector 58 adapted for connection with a female tubular connector of a respiratory component, such as a nebuliser. The mounting 52 includes a pair of diametrically opposed, rectangular, retaining openings 53 that are adapted to engage, and in particular receive with a snap fit, corresponding projections 23 provided on an exterior surface of the cylindrical housing 22 of the connection component 20. This engagement between the projections 23 and openings 53 mounts the connection component 20 onto the valve port component 50.

The tubular connector 58 of the valve port component 50 has the form of a male 22mm tubular connector, with a tapered external surface, which is adapted to be received within a female 22mm tubular connector of a respiratory component, such as a nebuliser, with an interference fit, thereby connecting the respiratory component to the respiratory connector 10. In addition, as shown most clearly in Figures 3 and 5, the valve component 50 includes an annular internal skirt that extends co-axially from the interior end of the tubular connector 58, and defines a cylindrical internal surface of reduced diameter relative to the internal surface of the tubular connector 58.

As shown most clearly in Figures 3 to 6, the seal component 56 extends over the base of the recess formed between the annular internal skirt of the valve port component 50 and the interior surface of the mounting 52. The seal component 56 therefore defines an annular valve seat that is engaged by the lower end of the cylindrical housing 22, when the connection component 20 and the valve port component 50 are connected, thereby providing a hermetic seal between the connection component 20 and the valve port component 50. In addition, the seal component 56 also extends over the outer surface, and upper end, of the annular internal skirt of the valve port component 50, and hence defines an upper sealing surface, which is inclined so that it faces inwardly towards the longitudinal axis of the valve port component 50.

The closed configuration of the respiratory connector 10 is shown in Figures 3 and 4. In this configuration, a lower portion of the tubular actuator member 46 projects from the lower end of the tubular connector 58, and is slidably engaged with the interior annular skirt of the valve port component 50. The upper sealing disc 42 is aligned with an upper surface of the inlet/outlet ports 24, such that the upper sealing disc 42 is not disposed within the fluid conduit that extends through the connection component 20. Similarly, the second pair of diametrically opposed struts 43, which separate the upper and lower sealing discs 42,44, are disposed within the recesses on either side of the fluid conduit.

The valve spring 30 acts to urge the upper sealing member 42, and hence the lower sealing member 44, towards the valve port component 50. The lower sealing disc 44 is therefore urged against the upper valve seat of the sealing component 56, as shown most clearly in Figure 4. The edges of the lower sealing disc 44 are rounded, and hence form an effective seal against the inclined upper sealing surface of the sealing component 50. In particular, the lower sealing disc 44 hermetically seals the interior of the valve port component 50 from the fluid conduit within the connection component 20. In addition, the lower sealing disc 44 is located substantially externally of the fluid conduit in the closed configuration. The flow of gas within the fluid conduit of the connection component 20 is schematically indicated by arrow 60 in Figures 3 and 4.

When the tubular connector 58 of the valve port component 50 is received within a female tubular connector of a respiratory component (not shown in the Figures), such as a nebuliser, the interior surface of the female tubular connector will engage the exterior surface of the tubular connector 58 of the valve port component 50 with an interference fit. In addition, formations on an interior surface of the female tubular connector will abut the tubular actuator member 46, and then cause the tubular actuator member 46 to move upwardly towards the connection component 20 into the open configuration, as shown in Figures 5 and 6, as the female tubular connector is fully engaged with the tubular connector 58 of the valve port component 50.

In the open configuration, the tubular actuator member 46 is disposed substantially externally of, but adjacent to, the fluid conduit through the connection component 20. The lower sealing disc 44 is disposed within the fluid conduit of the connection component 20, but its major surfaces, ie its upper and lower surfaces, are orientated parallel to the flow of gas through the fluid conduit, which is schematically indicated by arrows 60 in Figures 5 and 6. The lower sealing disc 44 therefore only occupies a small cross-sectional area of the fluid conduit. Furthermore, a flow path from the female tubular connector of the respiratory component, to the fluid conduit, is defined by the respiratory connector 10. This flow path is schematically indicated by arrow 62 in Figures 5 and 6, and extends through the tubular actuator member 46 and then a flow opening defined between the first pair of separator struts 47.

The first pair of diametrically opposed struts 47, which separate the lower seal disc 44 and the tubular actuator member 46, and also a lower part of the second pair of diametrically opposed struts 43, which separate the upper and lower sealing discs 42,44, are disposed within the recesses on either side of the fluid conduit. Furthermore, in this configuration, the valve spring 30 is compressed between the upper sealing disc 42 and the internal surface of the upper end of the cylindrical housing 22, such that a downward force is exerted on the valve member 40. However, the interference fit between the tubular connector 58 of the valve port component 50 and the female tubular connector of the respiratory component prevents movement of the valve member 40 back to the closed configuration, unless the tubular connector 58 is removed from the female tubular connector by a user.

The respiratory connector 10 also includes a cap 29 for sealing the open end of the valve port, and a connecting arm 28 that extends between the cap 29 and an external surface of the connection component 20, as shown most clearly in Figures 1 and 2. When the connector 10 is in its closed configuration, the cap 29 is engaged with the open end of the valve port so as to close the valve port The engagement of the cap 29 with the valve port is not required in order to seal the breathing circuit, but it will reduce the risk of foreign objects becoming deposited on the interior of the valve port, which could enter the breathing circuit when a nebuliser is next engaged with the respiratory connector 10. Furthermore, the cap 29 is adapted to prevent accidental depression of the tubular actuator member 46, when the respirator connector 10 is incorporated within a breathing circuit, but a nebuliser is not connected. In use, the cap 29 is disengaged from the open end of the valve port , before a nebuliser is engaged with the valve port and the respiratory connector 10 is switched to its open configuration.

In use, the respiratory connector 10 is connected to a breathing circuit by connecting a breathing tube (not shown in the Figures) to each of the inlet/outlet ports 24 in a conventional manner. The breathing circuit will generally be arranged such that gases intended for inhalation will flow along the fluid conduit of the respiratory connector 10, as shown by arrow 60 in Figure 3. As described above, the lower sealing disc 42 is resiliently urged against the inclined sealing surface of the seal component 56, so that fluid communication between the fluid conduit of the respiratory connector 10 and the valve port is prevented. In addition, the cap 29 is engaged with the valve port in this configuration.

When a user wishes to connect a nebuliser to the breathing circuit, the user disengages the cap 29 from the valve port. The lower end of the valve port is then received within the female tubular connector of the nebuliser, such that the formations on the interior surface of the female tubular connector abut a lower end of the tubular actuator member 46. Further insertion of the lower end of the valve port into the female tubular connector of the nebuliser will cause the valve member 40 to move from its closed configuration, as shown in Figures 3 and 4, to its open configuration, as shown in Figures 5 and 6. In the open configuration, gas and entrained medicament are able to flow from the nebuliser, though the tubular actuator member 46 and the flow openings defined between the first pair of separator struts 47, into the fluid conduit of the respiratory connector 10. In this way, gas and entrained medicament mix with the gases flowing through the fluid conduit, and are therefore delivered by the breathing circuit to the patient.

When the user wishes to disconnect the nebuliser from the breathing circuit, the user removes the lower end of the valve port from the female tubular connector of the nebuliser. This causes the valve member 40 to be moved by the valve spring 30 from the open configuration to the closed configuration, and the nebuliser is disengaged from the respiratory connector 10. Finally, the cap 29 is engaged with the valve port.

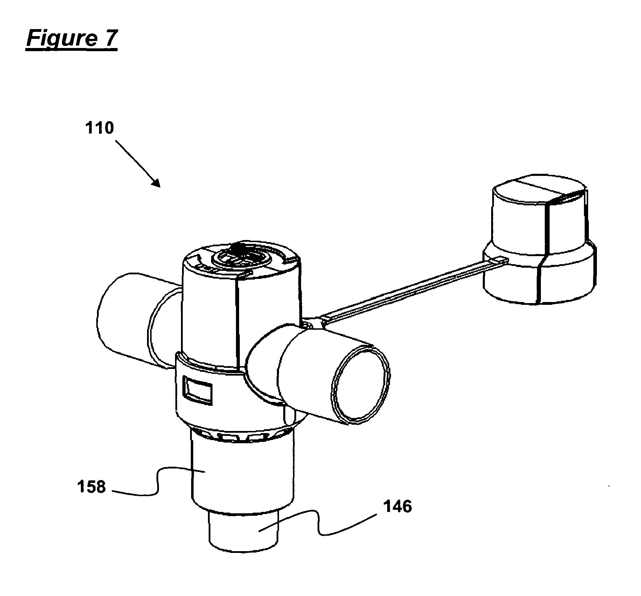

Figure 7 shows a second embodiment of a respiratory connector according to the invention, which is generally designated 110. This respiratory connector 110 is adapted to connect to a respiratory component, such as a nebuliser, having a male 22mm tubular connector. This respiratory connector 110 is identical to the first embodiment 10, save for the valve port 158 being of increased diameter, in particular having the form of a female 22mm tubular connector. In this case, the male 22mm tubular connector of the respiratory component engages the interior surface of the valve port 158 with an interference fit. In all other respects, the second embodiment 110 functions in an identical manner to the first embodiment 10. In particular, the interior surface of the male tubular connector of the respiratory component includes formations that are adapted to abut a lower end of the tubular actuator member 146 of the respiratory connector 110, while the tubular connector of the respiratory component is engaged with the valve port 158, such that the lower end of the tubular actuator member 146 is urged upwardly (as viewed in Figure 5) into the valve port 158.

## Claims

1. A respiratory connector (10) comprising first and second limbs defining a fluid conduit adapted for incorporation into a breathing circuit, a third limb defining a valve port (50) adapted for connection to a respiratory component, a valve member (40) movable relative to the valve port (50) between an open configuration in which the valve member (40) enables fluid communication between the valve port (50) and the fluid conduit, and a closed configuration in which the valve member (40) seals the valve port (50) from the fluid conduit, the respiratory connector (10) being **characterised in that** it comprises a fourth limb that houses means (30) for resiliently biasing the valve member (40) into the closed configuration.

2. A respiratory connector (10) as claimed in Claim 1, wherein the fourth limb extends from an opening in a part of a side wall of the fluid conduit that is substantially opposite that part of the side wall from which the third limb extends.

3. A respiratory connector (10) as claimed in Claim 1 or Claim 2, wherein the third and fourth limbs are aligned co-axially.

4. A respiratory connector (10) as claimed in any preceding claim, wherein the valve member (40) is received within the third limb defining the valve port (50), so that it is slidable relative to the valve port (50).

5. A respiratory connector (50) as claimed in Claim 4, wherein the valve member (40) is also received within the fourth limb, so that it is slidable relative to both the valve port (50) and the fourth limb.

6. A respiratory connector (10) as claimed in any preceding claim, wherein the resilient means (30) is interposed between an end wall of the fourth limb, and an end surface of the valve member (40).

7. A respiratory connector (10) as claimed in any preceding claim, wherein the valve membe (40) includes a sealing member that cooperates with a sealing surface of the valve port (50) so as to seal the valve port (50) from the fluid conduit in the closed configuration.

8. A respiratory connector (10) as claimed in Claim 7, wherein the sealing member is situated externally of the fluid conduit, so as not to impede flow through the fluid conduit, in the closed configuration.

9. A respiratory connector (10) as claimed in Claim 7 or Claim 8, wherein the sealing member is located within the fluid conduit in the open configuration.

10. A respiratory connector (10) as claimed in Claim 7 or Claim 8, wherein the sealing member is located within the chamber defined by the fourth limb of the respiratory connected (10) in the open configuration.

11. A respiratory connector (10) as claimed in any preceding claim, wherein the valve member (40) is adapted to be moved from the closed configuration to the open configuration by engagement of the respiratory component with a tubular connector (58) of the valve port (50).

12. A respiratory connector (10) as claimed in Claim 11, wherein the valve member (40) includes an actuator member (46) adapted to be engaged by a tubular connector the respiratory component, during its engagement with the tubular connector (58) of the valve port (50) such that the valve member (40) is moved from the closed configuration to the open configuration.

13. A respiratory connector (10) as claimed in Claim 12, wherein the valve member (40) includes a tubular actuator member (46).

14. A respiratory connector (10) as claimed in Claim 12 or Claim 13, wherein the respiratory connector is adapted for use with a respiratory component having a male tubular connector, and wherein a tubular connector (58) of the valve port (50) is adapted to engage an exterior surface of the male tubular connector with an interference fit, and the actuator member (46) is adapted to abut formations on the interior surface of the male tubular connector, and then be urged along the valve port (50) from the closed configuration to the open configuration.

15. A respiratory connector (10) as claimed in Claim 12 or Claim 13, wherein the respiratory connector (10) is adapted for use with a respiratory component having a female tubular connector, and wherein a tubular connector (58) of the valve port (50) is adapted to engage an interior surface of the female tubular connector with an interference fit, and the actuator members (46) is adapted to abut formations on the interior surface of the female tubular connector, and then be urged along the valve port (50) from the closed configuration to the open configuration.

## Patentansprüche

1. Beatmungstechnischer Verbinder (10), der ein erstes und ein zweites Glied aufweist, die eine Fluidleitung definieren, die zum Einschluss in ein Kreisteil ausgeführt ist, ein drittes Glied, das einen Ventilanschluss (50) definiert, der zum Verbinden mit einer beatmungstechnischen Komponente ausgeführt ist, ein Ventilelement (40), das relativ zu dem Ventilanschluss (50) zwischen einer offenen Konfiguration, in welcher das Ventilelement (40) die Fluidkommunikation zwischen dem Ventilanschluss (50) und der Fluidleitung ermöglicht, und einer geschlossenen Konfiguration, in welcher das Ventilelement (40) den Ventilanschluss (50) gegen die Fluidleitung abdichtet, beweglich ist, wobei der beatmungstechnische Verbinder (10) **dadurch gekennzeichnet ist, dass** er ein viertes Glied aufweist, in dem ein Mittel (30) zum federnden Vorspannen des Ventilelements (40) in die geschlossene Konfiguration untergebracht ist.

2. Beatmungstechnischer Verbinder (10) nach Anspruch 1, wobei das vierte Glied sich von einer Öffnung in einem Teil einer Seitenwand der Fluidleitung erstreckt, das dem Teil der Seitenwand, von dem sich das dritte Glied erstreckt, im Wesentlichen entgegengesetzt ist.

3. Beatmungstechnischer Verbinder (10) nach Anspruch 1 oder Anspruch 2, wobei das dritte und das vierte Glied koaxial aufeinander ausgerichtet sind.

4. Beatmungstechnischer Verbinder (10) nach einem der vorhergehenden Ansprüche, wobei das Ventilelement (40) in dem den Ventilanschluss (50) definierenden dritten Glied aufgenommen ist, so dass es im Verhältnis zum Ventilanschluss (50) verschiebbar ist.

5. Beatmungstechnischer Verbinder (50) nach Anspruch 4, wobei das Ventilelement (40) auch in dem vierten Glied aufgenommen ist, so dass es relativ zu dem Ventilanschluss (50) und zu dem vierten Glied verschiebbar ist.

6. Beatmungstechnischer Verbinder (10) nach einem der vorhergehenden Ansprüche, wobei das federnde Mittel (30) zwischen einer Endwand des vierten Glieds und einer Endfläche des Ventilelements (40) angeordnet ist.

7. Beatmungstechnischer Verbinder (10) nach einem der vorhergehenden Ansprüche, wobei das Ventilelement (40) ein Dichtungselement beinhaltet, das mit einer Dichtungsfläche des Ventilanschlusses (50) zusammenwirkt, um den Ventilanschluss (50) in der geschlossenen Konfiguration zur Fluidleitung abzudichten.

8. Beatmungstechnischer Verbinder (10) nach Anspruch 7, wobei das Dichtungselement sich außerhalb der Fluidleitung befindet, um in der geschlossenen Konfiguration den Durchfluss durch die Fluidleitung nicht zu behindern.

9. Beatmungstechnischer Verbinder (10) nach Anspruch 7 oder Anspruch 8, wobei das Dichtungselement sich in der offenen Konfiguration in der Fluidleitung befindet.

10. Beatmungstechnischer Verbinder (10) nach Anspruch 7 oder Anspruch 8, wobei das Dichtungselement sich in der offenen Konfiguration in der von dem vierten Glied des beatmungstechnischen Verbinders (10) definierten Kammer befindet.

11. Beatmungstechnischer Verbinder (10) nach einem der vorhergehenden Ansprüche, wobei das Ventilelement (40) ausgeführt ist, um durch Eingriff der beatmungstechnischen Komponente mit einem rohrförmigen Verbinder (58) des Ventilanschlusses (50) von der geschlossenen Konfiguration auf die offene Konfiguration bewegt zu werden.

12. Beatmungstechnischer Verbinder (10) nach Anspruch 11, wobei das Ventilelement (40) ein Stellelement (46) aufweist, das dafür ausgeführt ist, während seines Eingriffs mit einem rohrförmigen Verbinder (58) des Ventilanschlusses (50) von dem rohrförmigen Verbinder der beatmungstechnischen Komponente in Eingriff genommen zu werden, so dass das Ventilelement (40) aus der geschlossenen Konfiguration auf die offene Konfiguration bewegt wird.

13. Beatmungstechnischer Verbinder (10) nach Anspruch 12, wobei das Ventilelement (40) ein rohrförmiges Stellelement (46) beinhaltet.

14. Beatmungstechnischer Verbinder (10) nach Anspruch 12 oder Anspruch 13, wobei der beatmungstechnische Verbinder zur Verwendung mit einer beatmungstechnischen Komponente ausgeführt ist, die einen rohrförmigen Steckverbinder hat, und wobei ein rohrförmiger Steckverbinder (58) des Ventilanschlusses (50) dafür ausgeführt ist, in einer Presspassung mit einer Außenfläche des rohrförmigen Steckverbinders in Eingriff zu kommen, und das Stellelement (46) dafür ausgeführt ist, an Gebilden an der Innenfläche des rohrförmigen Steckverbinders in Anlage zu kommen und dann an dem Ventilanschluss (50) entlang von der geschlossenen Konfiguration auf die offene Konfiguration gedrängt zu werden.

15. Beatmungstechnischer Verbinder (10) nach Anspruch 12 oder Anspruch 13, wobei der beatmungstechnische Verbinder (10) zur Verwendung mit einer beatmungstechnischen Komponente ausgeführt ist, die einen rohrförmigen aufnehmenden Verbinder hat, und wobei ein rohrförmiger Verbinder (58) des Ventilanschlusses (50) dafür ausgeführt ist, in einer Presspassung mit einer Innenfläche des rohrförmigen aufnehmenden Verbinders in Eingriff zu kommen, und das Stellelement (46) dafür ausgeführt ist, an Gebilden an der Innenfläche des rohrförmigen aufnehmenden Verbinders in Anlage zu kommen und dann an dem Ventilanschluss (50) entlang von der geschlossenen Konfiguration auf die offene Konfiguration gedrängt zu werden.

## Revendications

1. Connecteur respiratoire (10) comprenant des premier et deuxième membres définissant un conduit de fluide adapté à l'incorporation dans un circuit de respiration, un troisième membre définissant un orifice de valve (50) adapté à la connexion à un composant respiratoire, un élément de valve (40) mobile par rapport à l'orifice de valve (50) entre une configuration ouverte dans laquelle l'élément de valve (40) permet une communication fluide entre l'orifice de valve (50) et le conduit de fluide, et une configuration fermée dans laquelle l'élément de valve (40) scelle l'orifice de valve (50) par rapport au conduit de fluide, le connecteur respiratoire (10) étant **caractérisé en ce qu'**il comprend un quatrième membre qui loge des moyens (30) pour solliciter élastiquement l'élément de valve (40) dans la configuration fermée.

2. Connecteur respiratoire (10) tel que revendiqué dans la Revendication 1, où le quatrième membre s'étend à partir d'une ouverture dans une partie d'une paroi latérale du conduit de fluide qui est substantiellement opposée à la partie de la paroi latérale à partir de laquelle s'étend le troisième membre.

3. Connecteur respiratoire (10) tel que revendiqué dans la Revendication 1 ou Revendication 2, où les troisième et quatrième membres sont alignés coaxialement.

4. Connecteur respiratoire (10) tel que revendiqué dans une quelconque revendication précédente, où l'élément de valve (40) est reçu au sein du troisième membre définissant l'orifice de valve (50) de manière à ce qu'il soit coulissable par rapport à l'orifice de valve (50).

5. Connecteur respiratoire (50) tel que revendiqué dans la Revendication 4, où l'élément de valve (40) est également reçu au sein du quatrième membre, de manière à ce qu'il soit coulissable par rapport à la fois à l'orifice de valve (50) et au quatrième membre.

6. Connecteur respiratoire (10) tel que revendiqué dans une quelconque revendication précédente, où les moyens élastiques (30) sont intercalés entre une paroi terminale du quatrième membre, et une surface terminale de l'élément de valve (40).

7. Connecteur respiratoire (10) tel que revendiqué dans une quelconque revendication précédente, où l'élément de valve (40) inclut un élément de scellement qui coopère avec une surface de scellement de l'orifice de valve (50) de manière à sceller l'orifice de valve (50) par rapport au conduit de fluide dans la configuration fermée.

8. Connecteur respiratoire (10) tel que revendiqué dans la Revendication 7, où l'élément de scellement est situé à l'extérieur du conduit de fluide, de manière à ne pas entraver l'écoulement à travers le conduit de fluide, dans la configuration fermée.

9. Connecteur respiratoire (10) tel que revendiqué dans la Revendication 7 ou Revendication 8, où l'élément de scellement est situé au sein du conduit de fluide dans la configuration ouverte.

10. Connecteur respiratoire (10) tel que revendiqué dans la Revendication 7 ou Revendication 8, où l'élément de scellement est situé au sein de la chambre définie par le quatrième membre du connecteur respiratoire (10) dans la configuration ouverte.

11. Connecteur respiratoire (10) tel que revendiqué dans une quelconque revendication précédente, où l'élément de valve (40) est adapté pour être déplacé de la configuration fermée à la configuration ouverte par mise en prise du composant respiratoire avec un connecteur tubulaire (58) de l'orifice de valve (50).

12. Connecteur respiratoire (10) tel que revendiqué dans la Revendication 11, où l'élément de valve (40) inclut un élément actionneur (46) adapté pour être mis en prise par un connecteur tubulaire du composant respiratoire, pendant sa mise en prise avec le connecteur tubulaire (58) de l'orifice de valve (50), de telle sorte que l'élément de valve (40) soit déplacé de la configuration fermée à la configuration ouverte.

13. Connecteur respiratoire (10) tel que revendiqué dans la Revendication 12, où l'élément de valve (40) inclut un élément actionneur tubulaire (46).

14. Connecteur respiratoire (10) tel que revendiqué dans la Revendication 12 ou Revendication 13, où le connecteur respiratoire est adapté à l'utilisation avec un composant respiratoire ayant un connecteur tubulaire mâle, et où un connecteur tubulaire (58) de l'orifice de valve (50) est adapté pour mettre en prise une surface extérieure du connecteur tubulaire mâle avec un ajustement avec serrage, et l'élément actionneur (46) est adapté pour buter contre des formations sur la surface intérieure du connecteur tubulaire mâle, et puis poussé le long de l'orifice de valve (50) de la configuration fermée à la configuration ouverte.

15. Connecteur respiratoire (10) tel que revendiqué dans la Revendication 12 ou Revendication 13, où le connecteur respiratoire (10) est adapté à l'utilisation avec un composant respiratoire ayant un connecteur tubulaire femelle, et où un connecteur tubulaire (58) de l'orifice de valve (50) est adapté pour mettre en prise une surface intérieure du connecteur tubulaire femelle avec un ajustement avec serrage, et l'élément actionneur (46) est adapté pour buter contre des formations sur la surface intérieure du connecteur tubulaire femelle, et puis poussé le long de l'orifice de valve (50) de la configuration fermée à la configuration ouverte.
